# FASCICULE DE BREVET EUROPEEN

(11) **EP 0 554 342 B1**
(45) Date de publication et mention de la délivrance du brevet: **19.07.1995**
(21) Numéro de dépôt: 91919480.3
(22) Date de dépôt: 22.10.1991
(51) Int. Cl.: A61B 5/22, G01L 19/08, G01D 7/12

(54) **PROCEDE ET DISPOSITIF DE TRANSDUCTION POUR PRODUIRE UN EFFET SONORE, TACTILE OU VISUEL**
UMWANDLUNGSVERFAHREN UND VORRICHTUNG ZUR ERZEUGUNG EINES AKUSTISCHEN, FÜHLBAREN ODER VISUELLEN EFFEKTES
PROCESS AND TRANSDUCER FOR PRODUCING A SOUND, TACTILE OR VISUAL EFFECT

(30) Priorité: 26.10.1990 FR 9013316
(43) Date de publication de la demande: 11.08.1993
(73) Titulaire: FERTIER, André, F-75020 Paris (FR)
(72) Inventeur: FERTIER, André, F-75020 Paris (FR)
(74) Mandataire: Casalonga, Axel
(86) Numéro de dépôt international: FR9100829
(87) Numéro de publication internationale: WO9207510

(56) Documents cités:
- EP-A- 0 200 555
- EP-A- 0 268 378
- WO-A-85/02255
- US-A- 3 995 492
- PATENT ABSTRACTS OF JAPAN, vol. 9, no. 259 (P-397)(1982) 17 Octobre 1985 ; JP-A-60 108 715

## Description

La présente invention concerne un procédé et un dispositif de transduction permettant d'obtenir un effet sonore, tactile ou visuel à partir de la mesure d'une grandeur physique, telle que la différence de pression exercée sur un objet ou le déplacement de cet objet.

Les dispositifs de transduction actuellement connus, appelés également transducteurs, sont utilisés dans des domaines techniques et scientifiques pour fournir des mesures précises d'une pression ou d'un déplacement pour satisfaire aux besoins exclusivement techniques et scientifiques. Un tel dispositif est décrit par exemple dans le document WO-A-8502255.

La présente invention a pour objet un procédé de transduction permettant de fournir des applications multiples dans des domaines variés, y compris des domaines non techniques.

L'invention a également pour objet un dispositif de transduction ou transducteur, adapté pour répondre à des besoins non exclusivement scientifiques ou techniques, en particulier dans des secteurs de l'art, de la pédagogie, de l'animation et de la thérapie.

Dans une première application, l'invention permet de fournir un transducteur qui, à partir d'une variation de la pression ou du déplacement, produit un effet sonore ou musicale. Le transducteur peut être réalisé sous forme d'un jouet pour enfants ou nouveaux-nés qui trouveront la joie de s'initier dans la production mélodique, ou sous forme d'objets décoratifs portables pour l'animation musicale ou la musicothérapie.

Dans une seconde application, l'invention vise un transducteur qui, à partir des variations de pression ou de déplacement, produit un effet de vibrations tactiles à l'aide par exemple d'une membrane vibrante, en vue notamment de satisfaire aux besoins de perception tactile des personnes malentendantes.

Dans une troisième application, l'invention fournit un transducteur pouvant servir de moyens de commande à distance notamment des appareils électroménagers, et des instruments de musique électroniques.

Selon un mode de réalisation de l'invention, le transducteur présente une dimension suffisamment faible pour être pris dans une main d'adulte ou d'enfant, de façon à ce que la main puisse exercer des pressions sur le transducteur pour qu'il produise des effets sonores, tactiles ou visuels.

Selon l'invention, le transducteur comprend au moins un capteur qui mesure une grandeur physique telle qu'une pression ou un déplacement et fournit un signal électrique analogique proportionnel à la valeur mesurée de la grandeur physique; un convertisseur analogique/numérique qui convertit le signal électrique analogique en un signal électrique numérique; un microprocesseur qui fait correspondre le signal numérique à un numéro d'ordre d'émission et transforme le numéro d'ordre d'émission en un code spécifique; une interface de sortie traduisant le code spécifique représentatif du numéro d'ordre d'émission en un second signal électrique analogique; et un moyen de production d'effets sonores, tactiles ou visuels commandé par le second signal électrique analogique.

Le transducteur de l'invention comprend avantageusement une enveloppe déformable en matière plastique souple. Une pression exercée sur l'enveloppe flexible se traduit par une déformation de l'enveloppe qui agit alors mécaniquement à son tour sur le capteur, lequel mesure la pression transmise par l'enveloppe flexible et déclenche le fonctionnement interne du transducteur pour émettre une note musicale, une vibration tactile ou un effet visuel tel que changement de couleur sur un écran.

De préférence, le haut-parleur pour la production du son, la membrane pour les vibrations tactiles ou l'écran pour les effets visuels peuvent être assemblés à l'enveloppe pour former un ensemble unique et compact. Auquel cas, lorsque le transducteur est pris dans la main, une pression exercée par les doigts sur l'enveloppe souple permet d'obtenir immédiatement soit une note musicale, soit une vibration tactile, soit un changement de couleur sur la partie de l'enveloppe où est installé le moyen de production d'effets correspondants.

Selon l'invention, le procédé de transduction comprend les étapes suivantes :
a) définir au moins une série de plusieurs intervalles de données numériques ou binaires et attribuer à chacun des intervalles un numéro d'ordre d'émission;
b) mesurer une grandeur physique, telle qu'une pression ou un déplacement et fournir un signal électrique analogique proportionnel à la valeur mesurée de la grandeur physique;
c) convertir le signal analogique en un signal numérique;
d) comparer la valeur du signal numérique avec la série d'intervalles de données numériques pour trouver le numéro d'ordre d'émission correspondant au signal numérique;
e) effectuer une seconde mesure en répétant les étapes b) et suivantes afin d'obtenir un second numéro d'ordre d'émission correspondant à un second signal numérique;
f) émettre un signal de commande spécifique au second numéro d'ordre d'émission si les valeurs numériques des premier et second signaux numériques sont différentes;
g) considérer la valeur du second signal numérique et le second numéro d'ordre comme la valeur d'un premier signal numérique et un premier numéro d'ordre et répéter les étapes e) et suivantes.

Le signal de commande émis peut être utilisé pour commander le moyen de production de son, de vibrations tactiles ou d'effets visuels.

Le procédé peut comprendre une étape de temporisation entre les étapes d) et e) de façon à ce que la répétition prévue à l'étape g) ait lieu après l'étape de temporisation.

De façon avantageuse, l'invention prévoit un seuil numérique minimum pour ne tenir compte que des valeurs numériques supérieures au seuil. Le seuil peut être différent de zéro.

Lorsque le signal numérique à l'étape d) est inférieur au seuil, au lieu d'exécuter les étapes e) et suivantes, le procédé renvoie à l'étape b) pour recommencer la mesure de la grandeur physique.

Lorsque le second signal numérique obtenu selon l'étape e) est inférieur au seuil, le procédé renvoie alors directement à l'étape a).

L'invention sera mieux comprise à l'étude de la description détaillée de deux modes de réalisation de l'invention pris à titre d'exemples nullement limitatifs et illustrés par des dessins annexés, sur lesquels :
la figure 1 est une vue schématique du transducteur de l'invention;
la figure 2 est un schéma synoptique d'un premier mode de fonctionnement du transducteur selon la figure 1, et
la figure 3 est un schéma sunoptique d'un second mode de fonctionnement du transducteur de la figure 1.

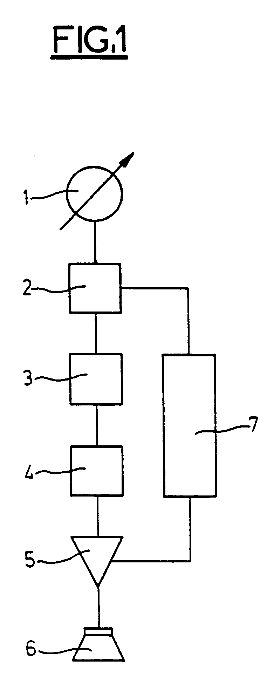

Tel qu'illustré sur la figure 1, le transducteur de l'invention comprend un capteur de pression 1 relié à un convertisseur analogique/numérique 2, un microprocesseur 3, un générateur sonore 4, un amplificateur 5 relié à un haut-parleur 6, et une source d'alimentation électrique 7 pour assurer le fonctionnement de l'ensemble.

Le microprocesseur 3 comprend une mémoire morte dans laquelle sont stockés des programmes de traitements spécifiques et une ou plusieurs séries d'intervalles numériques identifiés par leur numéro d'ordre, et une mémoire vive où sont effectués les traitements numériques des données.

Par exemple, pour une résolution de 8 bits, le convertisseur analogique/numérique traduit la pression mesurée par le capteur en un nombre binaire compris entre 0 et 255. Le programme contenu dans la mémoire morte du microprocesseur 3 permet de convertir le nombre binaire en codes compréhensibles par le générateur sonore 4. Les codes, également appelés numéro d'ordre d'émission, sont des numéros d'identification des intervalles numériques dans une série de données.

Si l'on veut produire une mélodie musicale selon une gamme pentatonique, la série d'intervalles numériques correspondante choisie comporte cinq intervalles utiles. Si le seuil minimum de pression à prendre en compte correspond à zéro, la plage numérique utile s'étend alors de 1 à 255. La définition d'une série de 5 intervalles sur cette plage utile signifie que pour chaque intervalle, la largeur numérique est égale à 255/5, c'est-à-dire 51. Les cinq intervalles sont classés dans l'ordre croissant par des numéros d'ordre variant de 0 à 4.

Pour une pression mesurée correspondant à un nombre binaire égal à 87 par exemple, le numéro d'ordre de l'intervalle correspondant est déterminé par 87 (valeur numérique de la pression mesurée)/51 (largeur d'un intervalle), le rapport donnant 1 comme numéro d'ordre.

Le générateur sonore 4 peut générer une gamme pentatonique de notes musicales correspondant chacune à un numéro d'ordre d'intervalle. Par exemple, on peut faire correspondre le numéro d'ordre 0 à la note la, 1 à do, 2 à ré, 3 à mi et 4 à sol.

Ainsi, la note générée par le générateur sonore 4 est fonction du numéro d'ordre de l'intervalle où se situe la valeur binaire de la pression mesurée par le capteur 1. Dans l'exemple précédent, la valeur binaire 87 de la pression se situant dans l'intervalle dont le numéro d'ordre est 1 se traduit par la note do.

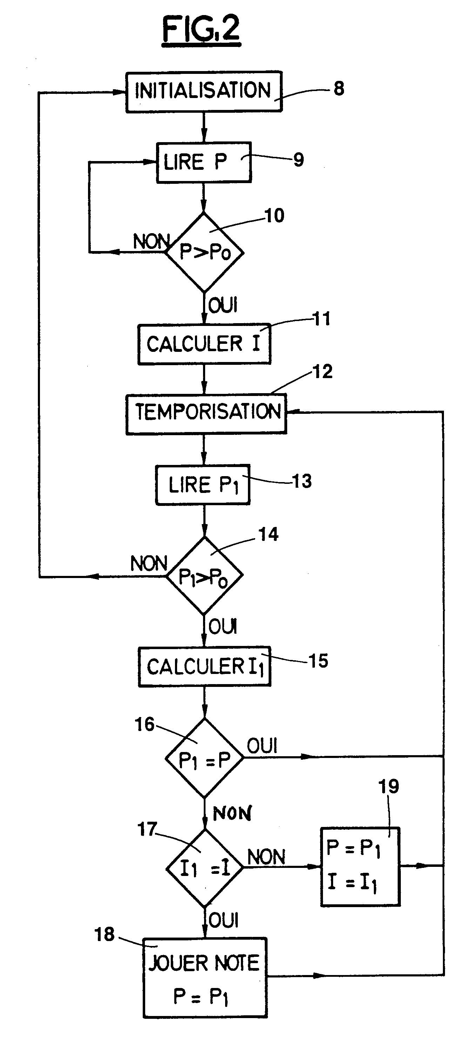

Le fonctionnement du transducteur de l'invention peut être schématisé comme suit (figure 2) :
une première étape 8 qui consiste à initialiser l'ensemble afin notamment de définir le nombre de notes disponibles pour être jouées par le transducteur. Ce qui revient à définir la largeur des intervalles dans les séries d'intervalles numériques. Comme montré précédemment, pour une résolution de 8 bits et 5 notes disponibles, la largeur d'un intervalle est de 51. Pour un nombre de notes égal à 12, la largeur d'un intervalle est alors de 225/12 c'est-à-dire 21. Les numéros d'ordre des intervalles sont alors de 0 à 11 pour les 12 notes disponibles générées par le générateur sonore 4.

L'étape suivante 9 est la lecture de la pression mesurée par le capteur 1 qui fournit un signal électrique analogique correspondant. Le signal est converti en signal numérique par le convertisseur analogique/numérique 2. La valeur du signal numérique P de la pression est comparée (l'étape 10) à une valeur de seuil minimal P0 dans la mémoire vive du microprocesseur 3.

Si P est inférieur ou égal à P0, on retourne alors à l'étape 9 pour recommencer la lecture du capteur 1. Si P est supérieur à P0, le microprocesseur 3 calcule alors le numéro d'ordre I de l'intervalle où se trouve la valeur de P. Cette étape est référencée 11.

Après l'étape 11, le transducteur passe dans une phase de temporisation 12 avant de lire à nouveau le capteur 1 (l'étape 13). La pression lue est convertie en valeur d'un signal numérique P1 par le convertisseur analogique/numérique 2 et est comparée ensuite, à l'étape 14, avec le seuil minimum P0 dans la mémoire vive du microprocesseur 3.

Si P1 est inférieur ou égal à P0, cela signifie que la pression mesurée correspondante au signal numérique P1 n'est pas suffisamment importante pour être prise en compte par le transducteur, on retourne alors à l'étape 8 d'initialisation pour recommencer les étapes précédemment décrites.

Si P1 est supérieur à P0, le microprocesseur 3 calcule alors à l'étape 15, le numéro d'ordre I1 de l'intervalle où se situe la valeur binaire de P1.

L'étape suivante, référencée 16, consiste à comparer la valeur numérique P1 de la pression significative lue à l'étape 14 avec la valeur numérique P de la pression lue à l'étape 9.

Si P1 est égal à P, c'est-à-dire la pression n'a pas changée entre les deux lectures des étapes 9 et 13, on retourne alors à la phase de temporisation 12 pour recommencer l'étape 13 de lecture.

Si P1 est différent de P, l'étape suivante 17 consiste alors à comparer les numéros d'ordre I et I1 calculés respectivement aux étapes 11 et 15.

Lorsque I1 est égal à I, c'est-à-dire les deux pressions correspondante aux signaux numériques P et P1 restent dans le même intervalle, le microprocesseur 3 délivre alors un signal de commande représentatif du numéro d'ordre I pour commander le générateur sonore 4 à délivrer une note correspondante. La note générée par le générateur sonore 4 et ensuite amplifiée dans l'amplificateur 5 pour être diffusée par le haut-parleur 6 (l'étape 18). A la fin de l'étape 18, la valeur de P est remplacée par la valeur de P1 et on revient à l'étape de temporisation 12 pour recommencer la lecture de pression à l'étape 13.

Si les numéros d'ordre I1 et I sont différents, on remplace alors les valeurs initiales de P et de I par les valeurs de P1 et de I1 à l'étape 19 et on retourne à la phase de temporisation 12, c'est-à-dire il faudrait attendre la stabilisation de la pression dans un même intervalle pour pouvoir émettre un signal de commande au générateur sonore 4.

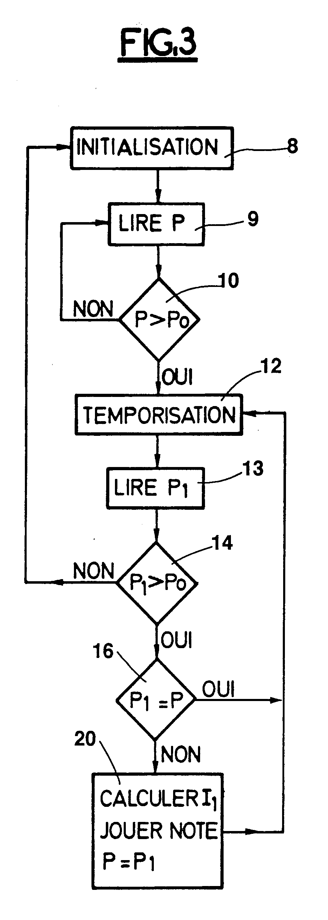

Selon une variante de l'invention telle qu'illustrée sur la figure 3, les étapes identiques à celles de la figure 2 comportent les mêmes références. Selon cette variante, il n'est pas nécessaire de calculer le numéro d'ordre I après l'étape 10. Il suffit de calculer le numéro d'ordre I1 de l'intervalle où se situe la pression lue (signal numérique P1) à l'étape 13 lorsque P1 est différent de P mesuré à l'étape 9. L'étape où l'on calcule I1 est référencée 20. A cette même étape 20, le microprocesseur 3 envoie un signal de commande correspondant au numéro d'ordre I1 au générateur sonore 4. La valeur P est remplacée par la valeur P1 avant de retourner à l'étape de temporisation 12 pour recommencer la lecture du capteur à l'étape 13.

Le mode de réalisation illustré sur la figure 3 diffère de celui illustré sur la figure 2 en ce qu'il n'est pas nécessaire d'attendre la stabilisation de la pression dans' un même intervalle pour délivrer un signal de commande, c'est-à-dire la suppression de la boucle constituée par des étapes 17, 19, 12, 13, 14, 15, 16 sur la figure 2.

Dans la pratique, le transducteur est de préférence noyé dans un matériau élastique tel que l'élastomère sous une forme facile à être prise dans la main. Lorsqu'on exerce une pression sur le matériau élastique, le capteur 1 mesure la pression. Si on libère la pression tout de suite, aucun son ne sort du transducteur. Cela permet d'éviter qu'une chute accidentelle du transducteur provoque une émission sonore non souhaitée.

En revanche si on fait varier la pression de façon dynamique, le transducteur de l'invention émet des notes musicales différentes. On peut faire ainsi varier intentionnellement les notes en contrôlant la pression exercée sur la partie élastique du transducteur.

Il en est de même pour des applications supplémentaires. Le signal de commande peut être utilisé également pour faire varier la fréquence de vibration d'une membrane destinée par exemple à être perçue tactilement par une personne malentendante. Cette personne peut faire varier la pression sur le transducteur et ressentir la vibration de la membrane qui en résulte.

L'invention peut également fournir un moyen de commande à distance, grâce à une variation de pression, d'appareils électroniques.

## Revendications

1. Procédé de transduction comprenant les étapes suivantes :
a) définir un certain nombre d'intervalles de donnée numériques ou binaires en leur attribuant des numéros d'ordre (I, I1) correspondants;
b) mesurer une grandeur physique, telle qu'une pression ou un déplacement et fournir un signal électrique analogique proportionnel à la grandeur mesurée;
c) convertir le signal analogique en un signal numérique (P);
d) comparer la valeur du signal numérique (P) avec les intervalles pour trouver le numéro d'ordre d'émission (I) correspondant au signal numérique;
e) effectuer une seconde mesure en répétant les étapes b), c) et d) pour obtenir un second numéro d'ordre (I1) correspondant à un second signal numérique (P1);
f) émettre un signal de commande spécifique au second numéro d'ordre (I1) si la valeur du signal numérique (P) mesurée en premier lieu est différente de la valeur du signal numérique (P1) de la même grandeur physique mesurée en second lieu;
g) remplacer la valeur du signal numérique (P) mesurée en premier lieu et le numéro d'ordre (I) correspondant par la valeur du signal numérique (P1) mesurée en second lieu et son numéro d'ordre correspondant (I1) et répéter les étapes e) et suivantes.

2. Procédé de transduction selon la revendication 1, caractérisé par le fait qu'à l'étape f), l'émission du signal de commande a lieu si le second numéro d'ordre (I1) est égal au premier numéro d'ordre (I).

3. Procédé de transduction selon la revendication 1 ou 2, caractérisé par le fait qu'il comprend une étape de temporisation (12) entre les étapes d) et e), et qu'à la fin de l'étape f) ou lorsque les valeurs mesurées des signaux numériques (P) et (P1) sont égales, on retourne à l'étape de temporisation avant de continuer les étapes e) et suivantes.

4. Procédé de transduction selon l'une des revendications précédentes, caractérisé par le fait que si la valeur du signal numérique (P) mesurée en premier lieu est inférieure ou égale à un seuil minimum (P0), on revient à l'étape b).

5. Procédé de transduction selon l'une des revendications précédentes, caractérisé par le fait que si la valeur du signal numérique (P1) mesurée en second lieu est inférieure ou égale à un seuil minimum (P0), on revient à l'étape a).

6. Procédé de transduction selon l'une des revendications précédentes, caractérisé par le fait qu'il comprend une étape de génération et d'émission des notes musicales correspondant au signal de commande émis à l'étape f).

7. Procédé de transduction selon l'une des revendications précédentes, caractérisé par le fait qu'il comprend une étape supplémentaire de production d'effets visuels, de vibrations tactiles et/ou d'ondes électromagnétiques de commande, correspondant au signal de commande émis à l'étape f).

8. Dispositif de transduction comprenant au moins un capteur (1) qui mesure une grandeur physique telle qu'une pression, un convertisseur analogique/numérique (2), un micro-processeur (3) et un moyen de production d'effets sonores, tactiles et/ou visuels commandé par le micro-processeur, le capteur fournissant un signal électrique analogique proportionnel à la valeur mesurée de la grandeur physique, le convertisseur transformant le signal analogique du capteur en un signal numérique, le microprocesseur permettant de faire correspondre le signal numérique à un numéro d'ordre d'émission (I, I1), le dispositif comprenant une interface de sortie qui transforme le numéro d'ordre d'émission en un signal électrique de commande du moyen de production d'effets sonores, tactiles et/ou visuels et une enveloppe en matériau élastique couvrant au moins le capteur et sur laquelle on exerce la grandeur physique, caractérisé par le fait que ledit dispositif fonctionne selon le procédé tel que défini dans l'une des revendications précédentes.

## Claims

1. Transducing process comprising the following steps:
a) defining a certain number of intervals of digital or binary data and assigning them respective rank number (I,I1);
b) measuring a physical magnitude such as a pressure or a displacement and producing an analog electrical signal proportional to the measured magnitude;
c) converting the analog signal into a digital signal (P);
d) comparing the value of the digital signal (P) with the intervals to find the rank number (I) corresponding to the digital signal;
e) carrying out a second measurement by repeating steps b),c) and d) to obtain a second rank number (I1) corresponding to a second digital signal (P1);
f) sending a command signal specific to the second rank number (I1) if the digital signal value (P) measured first is different than the digital signal value (P1) of the same physical magnitude measured second;
g) replacing the value of the digital signal P measured first and the corresponding rank number (I) by the value of of the digital signal P1 measured second and its corresponding rank number (I1) and repeat steps e) onwards.

2. Transducing process according to claim 1 characterized in that in step f) the command signal is sent if the second rank number (I1) is equal to the first rank number (I).

3. Transducing process according to claim 1 or claim 2 characterized in that it comprises a time-delay step (12) between steps d) and e) and in that at the end of step f) or if the measured values of the digital signals (P) and (P1) are equal the procedure returns to the time-delay step before continuing steps e) onwards.

4. Transducing process according to any one of the preceding claims characterized in that if the value of the digital signal (P) measured first is less than or equal to a minimum threshold (P0)the process returns to step b).

5. Transducing process according to any one of the preceding claims characterized in that if the value of the digital signal (P1) measured second is less than or equal to a minimum threshold (P0) the process returns to step a).

6. Transducing process according to any one of the preceding claims characterized in that it comprises a step of generating and sending musical notes corresponding to the command signal sent in step f).

7. Transducing process according to any one of the preceding claims characterized in that it comprises a further step of producing visual effects, tactile vibrations and/or electromagnetic command waves corresponding to the command signal sent in step f).

8. Transducer comprising at least one sensor (1) which measures a physical magnitude such as a pressure , an analog/digital converter (2), a microprocessor (3) and means of producing sound, tactile and/or visual effects commanded by the microprocessor , the sensor supplying an analog electrical signal proportional to the measured value of the physical magnitude, the converter converting the analog signal from the sensor into a digital signal, the microprocessor establishing the correspondence between the digital signal and an rank number (I,I1), the device comprising an output interface which converts the rank number into an electrical command signal for the sound , tactile and/or visual effect producing means and an elastic material envelope covering at least the sensor and on which the physical magnitude is exerted, characterized in that said device operates by the process as defined in any one of the preceding claims.

## Patentansprüche

1. Umwandlungsverfahren, bestehend aus den folgenden Verfahrensschritten:
a) Festlegung einer bestimmten Anzahl vor vorgegebenen numerischen oder binären Intervallen mit gleichzeitiger Zuteilung entsprechender Ordnungszahlen (I, I₁);
b) Messung einer physikalischen Größe wie eines Druckes oder einer Verschiebung unter gleichzeitiger Bildung eines elektrischen Analogsignals, welches proportinal zu der gemessenen Größe ist;
c) Umwandlung des Analogsignals in ein numerisches Signal (P);
d) Vergleichen des Wertes des numerischen Signals (P) mit den festgelegten Intervallen, um auf diese Weise jene Ordnungszahl (I) zu finden, die dem jeweiligen numerischen Signal entspricht;
e) Durchführung einer zweiten Messung unter Wiederholung der Verfahrensschritte b), c) und d), um auf diese Weise eine zweite Ordnungszahl (I₁) zu finden, welche einem zweiten numerischen Signal (P₁) entspricht;
f) Abgabe eines bestimmten Steuersignals bei der zweiten Ordnungszahl (I₁), falls der zuerst gemessene Wert des numerischen Signals (P) sich von dem in dem folgenden gemessenen Wert des numerischen Signals (P₁) derselben physikalischen Größe unterscheidet, und
g) Ersetzen des zuerst gemessenen Wertes des numerischen Signals (P) und der jeweiligen Ordnungszahl (I) durch den in dem folgenden gemessenen Wert des numerischen Signals (P₁) und der entsprechenden Ordnungszahl (I₁), worauf die Verfahrensschritte e) und folgende wiederholt werden.

2. Umwandlungsverfahren nach Anspruch 1, dadurch **gekennzeichnet**, daß bei dem Verfahrensschritt f) zusätzlich die Abgabe eines Steuersignals erfolgt, falls die zweite Ordnungszahl (I₁) gleich wie die erste Ordnungszahl (I) ist.

3. Umwandlungsverfahren nach Anspruch 1 oder 2, dadurch **gekennzeichnet**, daß zwischen den Verfahrensschritten d) und e) ein Verzögerungsintervall (12) vorgesehen ist, und daß am Ende des Verfahrensschrittes f) oder bei Gleichheit der gemessenen Werte der numerischen Signale (P) und (P₁) eine Rückkehr zu dem Verzögerungsintervall erfolgt, bevor eine Fortführung der Verfahrensschritte e) und folgende stattfindet.

4. Umwandlung des Verfahrens nach einem der vorangegangenen Ansprüche, dadurch **gekennzeichnet**, daß eine Rückkehr zu dem Verfahrensschritt b) erfolgt, falls der zuerst gemessene Wert des numerischen Signals (P) kleiner oder gleich einem minimalen Schwellwert (P_{O}) ist.

5. Umwandlungsverfahren nach einem der vorangegangenen Ansprüche, dadurch **gekennzeichnet**, daß eine Rückkehr zu dem Verfahrensschritt a) erfolgt, falls der in dem folgenden gemessene Wert des numerischen Signals (P₁) kleiner oder gleich einem minimalen Schwellwert (P_{O}) ist.

6. Umwandlungsverfahren nach einem der vorangegangenen Ansprüche, dadurch **gekennzeichnet**, daß zusätzlich ein Verfahrensschritt zur Erzeugung und Angabe von musikalischen Tönen vorgesehen ist, welche dem während des Verfahrensschrittes f) abgegebenen Steuersignal entsprechen.

7. Umwandlungsverfahren nach einem der vorangegangenen Ansprüche, dadurch **gekennzeichnet**, daß ein zusätzlicher Verfahrensschritt zur Erzeugung von visuellen Effekten, von ertastbaren Vibrationen und/oder von elektromagnetischen Wellen vorgesehen ist, welche dem während des Verfahrensschrittes f) abgegebenen Steuersignal entsprechen.

8. Umwandlungsvorrichtung, mit wenigstens einem eine physikalische Größe wie den Druck messenden Sensor (1), einem analog/numerischen Wandler (2), einem Mikroprozessor (3) sowie einer von dem Mikroprozessor gesteuerten Einrichtung zur Erzeugung akustischer fühlbarer und/oder visueller Effekte, wobei der Sensor ein analoges elektrisches Signal abgibt, welches proportional zu dem gemessenen Wert der physikalischen Größe ist, wobei der Wandler eine Umwandlung des analogen Signals des Sensors in ein numerisches Signal durchführt, wobei der Mikroprozessor eine Ordnungszahl (I, I₁) in Anhängigkeit des numerischen Signals festlegt, wobei die Vorrichtung zusätzlich eine Ausgangsschnittstelle besitzt, welche die abgegebene Ordnungszahl in ein elektrisches Steuersignal für die den akustischen, fühlbaren und/oder visuellen Effekt erzeugende Einrichtung umwandelt, und wobei wenigstens der die physikalische Größe erfassende Sensor mit einer aus Plastikmaterial bestehenden Hülle umgeben ist, dadurch **gekennzeichnet**, daß die betreffende Vorrichtung nach dem Verfahren entsprechend einer der vorangegangenen Ansprüche arbeitet.
